Europäisches Patentamt

**European Patent Office**

Office européen des brevets

(19)

(11) **EP 1 588 721 A1**

(12) **EUROPÄISCHE PATENTANMELDUNG**

(43) Veröffentlichungstag:
26.10.2005 Patentblatt 2005/43

(51) Int Cl.⁷: **A61L 2/18**, C11D 3/00

(21) Anmeldenummer: **04009361.9**

(22) Anmeldetag: **21.04.2004**

(84) Benannte Vertragsstaaten:
**AT BE BG CH CY CZ DE DK EE ES FI FR GB GR HU IE IT LI LU MC NL PL PT RO SE SI SK TR**
Benannte Erstreckungsstaaten:
**AL HR LT LV MK**

(71) Anmelder: **Predinal GmbH**
**24568 Kaltenkirchen (DE)**

(72) Erfinder:
• **Zerling, Wolfgang**
  **24568 Kaltenkirchen (DE)**
• **Baier, Michael, Dr.**
  **10435 Berlin (DE)**
• **Brill, Holger, Dr.**
  **22846 Norderstedt (DE)**

(54) **Mittel zur Inaktivierung infektiöser Prionen auf Flächen, Instrumenten und in kontaminierten Flüssigkeiten**

(57) Beschrieben wird ein Mittel zur Inaktivierung von infektiösen Prionen (Erreger von vCJK, BSE, Kuru etc.), bestehend aus synergistisch wirksamen Kombinationen von anionischen Tensiden mit bestimmten anorganischen und organischen Säuren, wegen material-verträglicher Eigenschaften, geeignet zur Anwendung auf kontaminierten Instrumenten, Geräten, Flächen und in Flüssigkeiten.

**Beschreibung**

Eine bisher unbekannte Bedrohung stellt - seit dem Auftreten von BSE und der nachgewiesenen Übertragbarkeit dieser Tierkrankheit auf den Menschen - die neue Variante der Creutzfeldt-Jacob-Krankheit (vCJK)dar. Der Erreger der Transmissiblen Spongiformen Enzephalopathien (TSEs = CJK,vCJK,Kuru etc.) muss bezüglich der Inaktivierung/Desinfektion als eine außergewöhnliche Herausforderung verstanden werden.

Ursache der TSE-Erkrankungen sind fehlgefaltete Glykoproteine, die von ihrem Entdecker S. Prusiner als Prion(en) bezeichnet wurden (proteinaceus infectious only). Es konnte gezeigt werden, daß zur pathologischen Variante des Prions ein physiologisches Gegenstück existiert, das auch als Prion Isoform bezeichnet wird. In der Literatur findet sich die Bezeichnung $PrP^{Sc,BSE,CJD}$ als Kurzform für die pathologische Variante, während die physiologische Isoform mit $PrP^C$ (c = cell) abgekürzt wird.

Beide Varianten des Prion-Proteins resultieren aus einem gemeinsamen Vorläuferprotein, das nach Reifung ein Molekulargewicht von 33-35 kd hat. Die Wandlung in das infektiöse Prion erfolgt - aus noch nicht bekannten Gründen - durch Konformationsänderung der Sekundärstruktur des Proteins, wobei alpha-Helices in die beta-Faltblattstruktur umgewandelt werden. Diese Wandlung geht einher mit einer deutlichen Änderung der physikalisch-chemischen Eigenschaften des nun fehlgefalteten Proteins.

Die veränderten Eigenschaften werden in diagnostischen Untersuchungsverfahren zur Differenzierung zwischen infektiösen und physiologischen Proteinen genutzt.

Die infektiöse Form der Prionen zeigt aufgrund der Sekundärstruktur eine partielle Resistenz gegenüber Proteasen. Von einem Prion - Protein $PrP^{Sc}$ mit 33 - 35 kd bleiben nach Behandlung mit Proteinase K etwa 28 - 30 kd unter Beibehaltung der Infektiosität erhalten. Als Kurzbezeichnung für alle pathologischen Prion - Formen wird daher auch häufig $PrP^{res}$ (res = resistant) verwendet. Die Prion Isoform $PrP^c$ (c = cell) wird hingegen vom Enzym vollständig verdaut.

Aus der beta-Faltblattstruktur erklärt sich u. a. auch die Widerstandsfähigkeit gegen hohe Temperaturen, UV-Strahlen, Säuren und Desinfektionsmittel sowie die Unlöslichkeit in Wasser, was einen Abbau im Organismus verhindert und Ablagerungen in Form von Fibrillen oder Amyloid-Plaques zur Folge hat. Diese Ablagerungen sind schließlich ursächlich für den Zelltod im Hirn. Die schwammförmige Feinstruktur des Hirngewebes läßt den Untergang der Zellen visuell erkennbar werden.

Von besonderer Bedeutung ist die Ausdünnbarkeit der Infektiosität fehlgefalteter Prionen im Tiermodell, dadurch ist der Grad der Infektiosität titrierbar, woraus sich die Möglichkeit herleitet, Agenzien unterschiedlichster Art auf eine inaktivierende Wirkung gegenüber pathogenen Prionen zu prüfen.

Gängige Desinfektionsmittel oder Inaktivierungsverfahren wie z. B. die Autoklavensterilisation sind entweder gar nicht oder nur in unbefriedigendem Ausmaß wirksam. Die Resistenz der fehlgefalteten, infektiösen Prionen gegenüber trockener Hitze übertrifft zudem die Überlebensfähigkeit bakterieller Sporen, so ist selbst bei kurzzeitiger Einwirkung von 600°C noch eine Restinfektiosität nachgewiesen worden.

Von Zobeley et al. konnte gezeigt werden, daß Prionen eine hohe Affinität zu Metalloberflächen aufweisen. Kleine Stahlstifte aus einem Material, wie es auch für chirurgische Instrumente verwendet wird, wurden mit dem Hirnhomogenat von Scrapie infizierten Mäusen kontaminiert, gereinigt und mit Formaldehyd behandelt und anschließend in das Gehirn von Indikatormäusen implantiert. Pro Stahlstift wurde eine verbliebene Infektiosität von ca. $10^5$ $LD_{50}$ errechnet.

Auf Grund des mit 1 - 2 Fällen pro 1 Million Einwohner pro Jahr sehr seltenen Vorkommens der Creutzfeld-Jakob-Krankheit(CJK) konnte bisher das Risiko einer iatrogenen Übertragung durch chirurgische Instrumente auf den Menschen vernachlässigt werden. Bei der chirurgischen Behandlung von CJK- Verdachtsfällen konnte das verwendete Gerät (OP-Besteck, Endoskope etc.) im Zweifelsfall entsorgt werden.

Mit dem Auftreten von vCJK, der BSE-Infektion des Menschen, hat sich diese Situation in Europa nachhaltig geändert. Anstelle weniger CJK-Fälle ist auch auf Grund epidemiologischer Hochrechnungen von einer unbekannten, ungleich größeren Anzahl infizierter Personen auszugehen, die über viele Jahre asymptomatisch sein werden, d. h. die nicht als vCJK- Verdachtsfälle eingeordnet werden können, dennoch als Träger der Krankheit diese, im Falle einer Behandlung mit chirurgischen Instrumenten oder Endoskopen, weiter verbreiten können.

Derzeit stehen als anerkannt wirksame Inaktivierungsmittel von $PrP^{res}$ lediglich Alkalien (z. B. Natronlauge; Kalilauge) und Hypochloritverbindungen (Chlorbleichlauge; Chlorabspalter)zur Verfügung. Beide Verbindungsklassen bewirken in hinreichend hoher Konzentration Hydrolyse oder Denaturierung des Proteinanteils im Prion.

Eine Verwendung dieser Chemikalien zur ständigen Desinfektion von chirurgischen Instrumenten oder Endoskopen verbietet sich, da aufgrund der aggressiven Eigenschaften mit einer baldigen Unbrauchbarkeit des Instrumentariums zu rechnen wäre.

Daraus ergab sich die Notwendigkeit nach Verbindungen oder Verbindungsklassen zu suchen, die einerseits im Hinblick auf ihre korrosiven Eigenschaften (Metallkorrosion, Spannungsrißkorrosionen etc.) beherrschbar sind und andererseits eine ausreichend inaktivierende Wirkung gegenüber pathogenen Prionen aufweisen.

Überraschend wurde nun gefunden, daß Gemische aus bestimmten anorganischen und organischen Säuren und Alkylsulfonaten bzw. -sulfaten und/oder Alkylarylsulfonaten diese Aufgabe zu lösen vermögen. Die Gemische wirken miteinander als Synergisten. Hingegen ist die Wirkung der Säuren ohne die anionischen Tenside und die der Tenside ohne Säuren sehr mangelhaft.

Nachfolgend aufgeführte Beispiele und Tabellen dienen der Erläuterung der vorliegenden Erfindung und dem Nachweis des Synergismus.

Gemäß F.C. Kull und P.C. Eisman, Applied Microbiology 9,538-41(1946) gilt ein Synergismus als nachgewiesen, wenn durch Anwendung der nachstehenden Berechnungsformel das Ergebnis F < 1 zustande kommt.

$$F = QA/Qa + QB/Qb \qquad\qquad (Gl.1)$$

wobei die Zeichen bedeuten:

| F < 1 | Synergismus |
|-------|-------------|
| F= 1  | Additive Wirksamkeit |
| F > 1 | Antagonismus |

Qa = Menge von A allein zum Endpunkt
Qb = Menge von B allein zum Endpunkt
QA = Menge von A in der Mischung mit B
QB = Menge von B in der Mischung mit A

Bei der Untersuchung der Synergisten als Einzelsubstanz konnte nicht immer ein Endpunkt gefunden werden, weil eine beliebige Konzentrationserhöhung wegen Methodik und/oder physikalischen Eigenschaften der jeweiligen Substanzen nicht realisierbar war. Die im Test verwendete höchste Konzentration wurde in diesen Fällen als die Konzentration zum Endpunkt angenommen und zur Berechnung in Gleichung (1) eingesetzt.

Zum Nachweis der Wirksamkeit und des synergistischen Effektes durch die erfindungsgemäßen Gemische wurden die Formulierungsbeispiele 1 - 9 verwendet.

Beispiele

| Beispiel 1 | Gew. Teile |
|------------|------------|
| Alkyl(C12)sulfat - Na -Salz | 20,00 |
| L(+)-Weinsäure DAB | 20,00 |
| Wasser entionisiert | 60,00 |
| Beispiel 2 | |
| Alkyl(C12)sulfat - Na -Salz | 20,00 |
| Wasser entionisiert | 80,00 |
| Beispiel 3 | |
| L(+)-Weinsäure | 40,00 |
| Wasser entionisiert | 60,00 |
| Beispiel 4 | |
| Alkyl (C8-14) sulfonat -Na- Salz | 20,00 |
| Ameisensäure (98%ig) | 40,00 |
| Wasser entionisiert | 40,00 |
| Beispiel 5 | |
| Alkylarylsulfonat-Na-Salz | 40,00 |
| Wasser entionisiert | 60,00 |

(fortgesetzt)

| Beispiel 1 | Gew. Teile |
|---|---|
| Beispiel 6 | |
| Ameisensäure | 40,00 |
| Wasser entionisiert | 60,00 |
| Beispiel 7 | |
| Alkyl(C12)sulfonat-Na-Salz | 20,00 |
| Ammidoschwefelsäure | 10,00 |
| Wasser entionisiert | 70,00 |
| Beispiel 8 | |
| Alkyl(C12)sulfonat-Na-Salz | 30,00 |
| Wasser entionisiert | 70,00 |
| Beispiel 9 | |
| Amidoschwefelsäure | 10,00 |
| Wasser entionisiert | 90,00 |

In vitro Prüfung

Das als Western- Blot bekannte in vitro Testverfahren, ermöglicht es, die Inaktivierung von Prionen über die Denaturierung / Zerstörung von PrP[res] qualitativ zu bestimmen.

Alle Substanzen bzw. Substanzgemische wurden zunächst mittels Blot-Methode untersucht. Konnte auf diesem Weg kein Abbau des infektiösen Hirnhomogenats durch Protease K festgestellt werden, so wurde die Formulierung als unwirksam verworfen.

Gemische die dazu führten, dass nach Einwirkung auf das infektiöse Material ein proteolytischer Abbau durch das Enzym wieder möglich war, wurden als potentiell wirksam eingestuft und einer quantitativen Untersuchung im Tiermodell zugeführt.

Ausgangsmaterial für den Test waren 100 µl - Ansätze eines 5%igen Hirnhomogenats. Zur Infektion wurde der Scrapie-Stamm 263 K verwendet.

Aus einem Hamsterhirn (mit ca. 1g Gewicht) ergibt sich ca. 1 mg Hirngewebe im 20 µl Ansatz mit einer durchschnittlichen Infektiosität von $10^6$ - $10^7$ infektiösen Einheiten ($LD_{50}$). Zur Kontrolle wird das Hirn von gesunden Hamstern verwendet.

Jeder Testansatz enthielt:

20 µl Hirnhomogenat
30 µl $H_2O$
50 µl Testsubstanz (diverse Konz.)

Jeder Kontrollansatz enthielt:

20 µl Hirnhomogenat
80 µl $H_2O$

Nach 30 minütiger Inkubation der Ansätze bei der gewünschten Temperatur von 37°C auf einem Schüttler, wurden diese zur Neutralisierung (Inaktivierung) der sauren Testsubstanzen auf pH 7,0 eingestellt. Der Kontrollansatz wurde mit dem gleichen Neutralisierungspuffer versetzt, der Neutralisierungspuffer für den Kontrollansatz wurde jedoch zuvor auf pH 7,0 eingestellt. Zur Neutralisierung der Säuren wurden durchschnittlich 2,5 mM - 1000 mM Tris-Base eingesetzt. Das Volumen aller auf pH 7.0 eingestellten Ansätze ergab 150 µl.

Jeweils 2 µl Ansatz (Testansatz bzw. Kontrollansatz) wurden anschließend mit 18 µl $H_2O$ und 2 µl Proteinase K- Stammlösung (1mg/ml) (Boehringer Mannheim) versetzt.

Der enzymatische Abbau durch Proteinase K erfolgte über 1 Stunde bei 37°C.

Als Kontrolle dienten Ansätze denen statt 2 µl Proteinase K 2 µl $H_2O$ zugegeben wurde und die ebenfalls 1 Stunde bei 37 C inkubiert wurden.

Abschließend wurde jeder Ansatz mit 5 µl Eiweiß- Solubilisierungspuffer versetzt. 20 µl von jedem Ansatz wurden zur SDS- Gelelektrophorese verwendet. Die Gele werden auf PVDF[1] - Membranen (0,45 µm) geblottet.

Analyse des Blots mit spezifischen Antikörpern:

Die Blotfolie wurde zur Vermeidung unspezifischer Bindungen auf der PVDF -Membran mit Milcheiweiß behandelt (Blockierung der Bindungsstellen) und diversen Spülvorgängen unterzogen, danach mit dem primären monoklonalen anti-Prion-protein Antikörper 3F4 inkubiert. Nach weiteren Vorbehandlungen und Spülungen wurde dann mit dem sekundären Antikörper - AP- konjugiertes anti Maus IgG aus der Ziege - inkubiert.

Nach erneutem Spülen wurde mit einem Lumineszenzpuffer inkubiert (Eine Lichtemission wird dadurch an den Stellen der Membran erzeugt, an denen sich die Prionproteine befinden). Nach dem Entfernen von überschüssiger Lösung wurde die Membran in eine Expositionsbox verbracht und auf Röntgenfilm entwickelt.

Eine Auswertung des Blots ist in Bild(1) wiedergegeben.

Daraus lässt sich zweifelsfrei erkennen, ob eine Wirkung durch Agenzienaktivität auf das infektiöse Material stattgefunden hat. Dennoch gibt dieses Verfahren keine sichere Auskunft über den Umfang einer Inaktivierung und die mögliche verbleibende Restinfektiosität.

Beispiel zum Test auf Prion-Inaktivierung. Western-Blot-Nachweis der Beseitigung von Proteinase K-resistentem Prion-Protein durch eine Testsubstanz , Verringerung der Wirkung durch kleinere Konzentrationen; (Spuren 1-4) Spur 5: Ansatz ohne Zugabe einer inaktivierenden Substanz

**Bild(1)**

Die in vitro Methode ermöglicht - nach internationalen Maßstäben - lediglich eine qualitative Aussage zur Wirksamkeit und gilt erst dann als validiert, wenn durch die quantitative in vivo Methode (Tiermodell) ein bestätigendes Ergebnis erzielt werden kann.

1 PVDF = Polyvinylidendifluorid

In vivo Prüfung (Tiermodell)

Zur Durchführung der Versuche im Tiermodell wurden Hamster mit 20µl der neutralisierten Proben und der Kontrollen intrazerebral beimpft (Lösungen wie bei der Blot-Methode). Für jede Probe wurde eine Zahl von mehr als 8 Hamstern verwendet, um zu einer statistisch belastbaren Aussage zu gelangen.

Tiere bei denen statt einer inaktivierenden Substanz nur Wasser verwendet wurde, starben im statistischen Mittel 86 Tage nach Inokulation an Scrapie.

Durch Anwendung der erfindungsgemäßen Gemische ist eine Verzögerung des Krankheitseintritts der Tiere von 125 Tagen und mehr erreichbar. Infektionsdosis und Inkubationszeit korrelieren miteinander, daher kann unter Einbeziehung entsprechender Titrationsexperimente für die Inkubationszeit eine damit einhergehende Titerreduktion angegeben werden Grafik(1).

Eine Verzögerung von z. B. 86 Tagen auf 125 Tage entspricht einer Titerreduktion von mehr als 4 logarithmischen Einheiten oder einer Reduktion der Infektiosität um 99,99%.

Die Infektiosität der Hirnhomogenate (Zahl der infektiösen Einheiten/Partikel) wurde durch Verdünnung zum Endpunkt bestimmt. Die gemittelten Ergebnisse aus den Verdünnungsreihen stehen halblogarithmisch in linearer Beziehung zu den Inkubationszeiten. Aus diesen Untersuchungen resultiert die graphische Darstellung Grafik(1) und die Regressionsfunktion (Gleichung 2), die zur Berechnung der Untersuchungsergebnisse aus den Tabellen 1-3 diente.

**TSE (Inkubationszeit vs. Infektiosität)**

$y = -0,1101x + 16,123$

$R^2 = 0,9954$

Log [Infektiöse Einheiten]

Inkubationszeit [d]

**Grafik(1)**

$$\text{(Gl. 2)} \qquad \log(ILD_{50}) = -0,1101 \times I_d + 16,123$$

$\log(ILD_{50}) =$      infektiöse Einheiten

$I_d =$      Inkubationszeit in Tagen

Regressions-Statistik:

| Bestimmtheitsmaß: | 0,9954 |
|---|---|

(fortgesetzt)

| Standardfehler : | 0,150116 | |
|---|---|---|
| Schnittpunkt : | 16,1227; | Standardfehler 0,35982 |
| Variable : | 0,1101; | Standardfehler 0,00333 |

Die gemittelten Ergebnisse der Beispielformulierungen 1 - 9 sind in den Tabellen 1 - 3 dargestellt. Die angegebenen Zahlenwerte bedeuten soweit nicht anders bezeichnet den Infektionstiter (log $LD_{50}$) der reduzierten infektiösen Dosis, deren Differenz zur Ausgangsdosis den Reduktionsfaktor ergibt.

## Tabelle 1

Konzentration [%]/ log (Infektiosität)

| Prüfmuster | log (inf. Dosis) | 0,5% | 1,0% | 2,0% | 5,0% |
|---|---|---|---|---|---|
| Beispiel 1 | 6,62 | 2,35 | | | |
| Beispiel 2 | 6,62 | 6,64 | 6,62 | 5,92 | 5,53 |
| Beispiel 3 | 6,62 | 6,60 | 6,55 | 6,32 | 5,41 |

## Tabelle 2

Konzentration [%] / log (Infektiosität)

| Prüfmuster | log (inf. Dosis) | 0,5% | 1,0% | 2,0% | 5,0% |
|---|---|---|---|---|---|
| Beispiel 4 | 6,62 | 3,68 | 2,48 | | |
| Beispiel 5 | 6,62 | 6,59 | 6,61 | 6,60 | 5,98 |
| Beispiel 6 | 6,62 | 6,63 | 6,52 | 5,89 | 5,61 |

## Tabelle 3

Konzentration [%]/ log (Infektiosität)

| Prüfmuster | log (inf. Dosis) | 0,5% | 1,0% | 2,0% | 5,0% |
|---|---|---|---|---|---|
| Beispiel 7 | 6,78 | 2,33 | | | |
| Beispiel 8 | 6,78 | 6,71 | 6,33 | 5,83 | 5,22 |
| Beispiel 9 | 6,78 | 6,60 | 6,55 | 6,32 | 5,57 |

Ein Ergebnis ist als ausreichend wirksam zu bewerten, wenn der Infektionstiter um 4 log-Stufen reduziert wurde, d. h. wenn eine 99,99%ige Reduktion der Infektiosität erreicht ist.

Aus Tabelle 1 folgt, dass mit der Säurekomponente aus Beispiel 3 und der Tensidkomponente aus Beispiel 2 keine ausreichende Wirksamkeit erzielt werden konnte. Formulierungsbeispiel 1 hingegen enthielt beide Komponenten und zeigte bereits bei 0,5%iger Konzentration eine ausreichende Inaktivierung infektiöser Prionen.

Setzt man diese Ergebnisse in die Formel von Kull und Eisman ein (Gl. 1), so erhält man

$$F = 0,5 \times 20/5 \times 40 + 0,5 \times 20/5 \times 20 = \underline{0,15}$$

Der Zahlenwert 0,15 erbringt somit einen eindeutigen Nachweis für das Vorliegen eines synergistischen Effektes.

Für Tabelle 2 mit den Beispielen 4-6 ergibt sich für F = 0,4 und für Tabelle 3 mit den Beispielen 7-9 für F = 0,076 als Zahlenwert.

Demzufolge ist die Wirksamkeit gegen pathogene Prionen und der synergistische Effekt der erfindungsgemäßen Gemische durch die dargestellten Ergebnisse bewiesen.

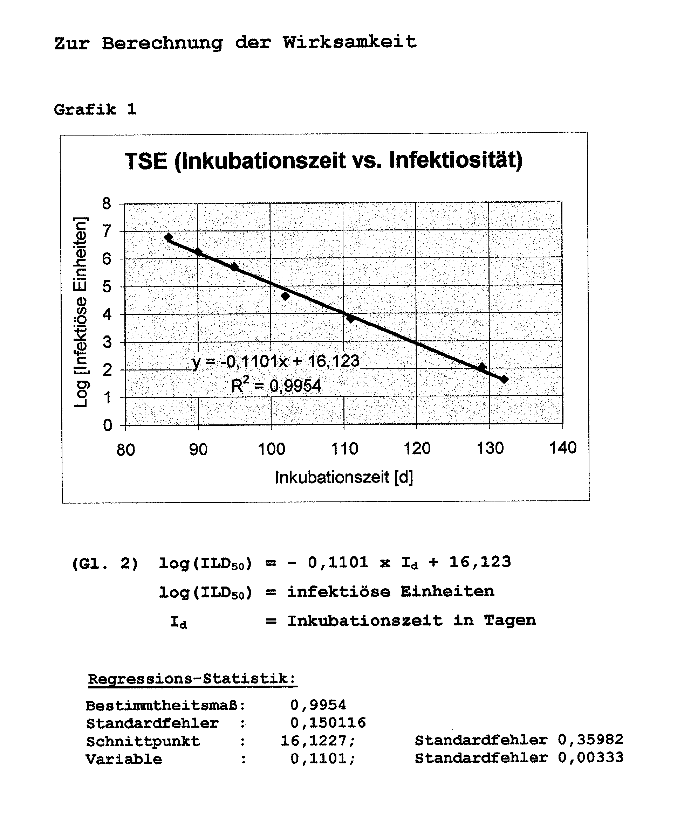

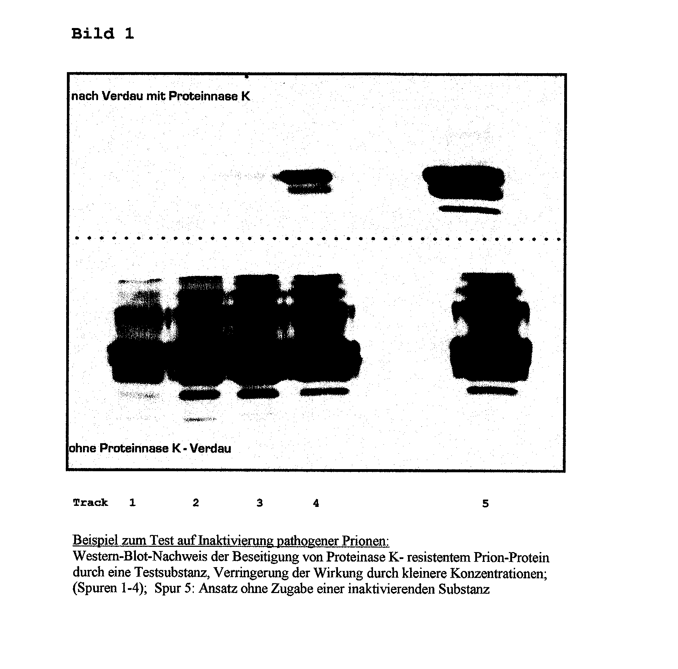

**Patentansprüche**

1. Mittel zur Verwendung als Inaktivierungsagens pathogener Prionen (PrP$^{res}$), zur Anwendung auf Flächen, Instrumenten medizinischer und technischer Einrichtungen sowie in kontaminierten Flüssigkeiten auf Basis eines synergistisch wirksamen Gemisches von bestimmten anorganischen und organischen Säuren mit anionaktiven Tensiden, die Hydrotropierungsmittel und nichtionogene Tenside als Netzmittel sowie primäre, sekundäre oder mehrwertige Alkohole als Lösungsmittel enthalten können, **dadurch gekennzeichnet, dass**

   a) sie synergistisch wirksame Gemische bestehend aus Amidosulfonsäure, o-Phosphorsäure, Ameisensäure, Glyoxylsäure, Essigsäure, Propionsäure, Milchsäure, Äpfelsäure, Weinsäure, Citronensäure, Gluconsäure, Cyclohexansulfaminsäure, Benzisothiazolin-2-on-S,S-dioxid (Saccharin als freie NH- acide Säure), Nitrilotriessigsäure, Ethylendiamintetraessigsäure und / oder deren Alkalioder Ammoniumsalze sowie organisch substituierte Phosphonsäuren wie 2-Phosphonobutan-1,2,4- tricarbonsäure, (1-Hydroxyethylen)-bis-phosphonsäure, Ethylendiamin-tetra-(methylenphosphonsäure), Diethylentriamin-penta-(methylenphosphonsäure), Amino-tris- (methylenphosphonsäure) und/oder deren Alkali- oder Ammoniumsalze einzeln oder im Gemisch miteinander in Kombination mit primären und/oder sekundären Alkyl(C8-C18)sulfonsäuren, Alkylarylsulfonsäuren und /oder deren Na-, K- oder Ammoniumsalzen, Alkyl(C8-C18)sulfaten sowie Alkylethersulfaten mit 1-3 EO-Gruppen und/oder Alkylarylethersulfaten mit 1-3 EO Gruppen und deren Na-, K- oder Ammoniumsalze enthalten sowie nichtionogene Tenside vom Typus der Alkylpolyethylenglykolether mit 3-11 EO-Gruppen enthalten können.

   b) sie Butylmonoglykolsulfat, Cumolsulfonat, Toluolsulfonat, Xyloylsulfonat als Natrium-, Kalium-oder Ammoniumsalze einzeln oder als Gemisch als Hydrotropierungsmittel und aliphatische Alkohole und/oder Glykole der Kettenlänge C2-C14 als Lösungsmittel einzeln oder als Gemisch enthalten können.

2. Desinfektionsmittel nach Anspruch 1 **dadurch gekennzeichnet, dass** das Gewichtsverhältnis der Alkylsulfate/ sulfonsäuren und/oder Alkylarylsulfate/sulfonsäuren und/oder Ethersulfate und deren Salze (A) mit den Säuren (B) einzeln oder im Gemisch miteinander im Verhältnis A:B = 1:99 und 99:1 liegen kann und deren Summe zwischen 5 und 60% bezogen auf das Gesamtgewicht des Desinfektionsmittelkonzentrats betragen kann.

3. Desinfektionsmittel nach Anspruch 1 **dadurch gekennzeichnet, dass** das Gewichtsverhältnis der Hydrotropierungsmittel und deren Salze, einzeln oder im Gemisch miteinander zwischen 5 und 60 Gew.% bezogen auf das Gesamtgewicht des Desinfektionsmittelkonzentrates betragen kann.

4. Desinfektionsmittel nach Anspruch 1 **dadurch gekennzeichnet, dass** das Gewichtsverhältnis der Alkohole einzeln oder im Gemisch miteinander zwischen 5 und 60 Gew.% bezogen auf das Gesamtgewicht des Desinfektionsmittelkonzentrates liegen kann.

5. Verwendung der Desinfektionsmittel nach einem der Ansprüche 1-4 zur Inaktivierung pathogener Prionen im Umfeld medizinischer und lebensmitteltechnischer Einrichtungen.

6. Desinfektionsmittel nach einem der Ansprüche 1-5 **dadurch gekennzeichnet, dass** das Mittel zusätzlich auch andere mikrobizide Wirkstoffe enthalten kann, um zur Desinfektion umfassend anwendbar zu sein.

7. Desinfektionsmittel nach einem der Ansprüche 1-6 **dadurch gekennzeichnet, dass** das Mittel zusätzlich Korrosionsinhibitoren enthalten kann.

8. Verwendung der Desinfektionsmittel nach einem der Ansprüche 1-7 in wässrigen, verdünnten Lösungen, die zwischen 0,1 und 60% des Desinfektionsmittelkonzentrats enthalten können.

Zur Berechnung der Wirksamkeit

Grafik 1

## TSE (Inkubationszeit vs. Infektiosität)

$$y = -0{,}1101x + 16{,}123$$
$$R^2 = 0{,}9954$$

(Gl. 2)  $\log(ILD_{50}) = -0{,}1101 \times I_d + 16{,}123$

$\log(ILD_{50})$ = infektiöse Einheiten

$I_d$ = Inkubationszeit in Tagen

Regressions-Statistik:

| | | | |
|---|---|---|---|
| Bestimmtheitsmaß: | 0,9954 | | |
| Standardfehler : | 0,150116 | | |
| Schnittpunkt : | 16,1227; | Standardfehler | 0,35982 |
| Variable : | 0,1101; | Standardfehler | 0,00333 |

## Bild 1

nach Verdau mit Proteinnase K

ohne Proteinnase K - Verdau

Track    1       2       3       4              5

Beispiel zum Test auf Inaktivierung pathogener Prionen:
Western-Blot-Nachweis der Beseitigung von Proteinase K- resistentem Prion-Protein
durch eine Testsubstanz, Verringerung der Wirkung durch kleinere Konzentrationen;
(Spuren 1-4); Spur 5: Ansatz ohne Zugabe einer inaktivierenden Substanz

<table>
<tr><td colspan="2">Europäisches<br>Patentamt</td><td>EUROPÄISCHER RECHERCHENBERICHT</td><td>Nummer der Anmeldung<br>EP 04 00 9361</td></tr>
</table>

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| X | US 2004/052833 A1 (PRUSINER STANLEY B ET AL) 18. März 2004 (2004-03-18) * Absätze [0003], [0024], [0047], [0048], [0107], [0115], [0127], [0149], [0354]; Ansprüche * | 1-8 | A61L2/18 C11D3/00 |
| | ----- | | |
| X | US 6 162 371 A (COYLE-REES MARGARET ET AL) 19. Dezember 2000 (2000-12-19) * Spalte 1, Zeilen 11,12 * * Ansprüche 1,5,11,12 * | 1-4,6-8 | |
| | ----- | | |
| X | US 4 247 408 A (IMAMURA TETSUYA ET AL) 27. Januar 1981 (1981-01-27) * Spalte 2, Zeilen 10,11,34-38; Ansprüche 2,3 * | 1-4,6-8 | |
| | ----- | | |
| X | US 5 280 042 A (LOPES JOHN A) 18. Januar 1994 (1994-01-18) * Spalte 3, Zeile 20 * * Spalte 5, Zeile 25 * * Spalte 8, Zeilen 53,54 * * Anspruch 1 * | 1-4,6-8 | **RECHERCHIERTE SACHGEBIETE (Int.Cl.7)** A61L C11D |
| | ----- | | |
| X | GB 896 159 A (SOFNOL LTD) 9. Mai 1962 (1962-05-09) * Seite 1, Zeilen 72-80 * * Seite 2, Zeilen 8-10 * * Ansprüche 4,12 * | 1-4,6-8 | |
| | ----- | | |

~~Der vorliegende Recherchenbericht wurde für alle Patentan~~sprüche erstellt

| Recherchenort | Abschlußdatum der Recherche | Prüfer |
|---|---|---|
| München | 8. November 2004 | Nissen, V |

# EP 1 588 721 A1

**Europäisches**
**Patentamt**

---

## GEBÜHRENPFLICHTIGE PATENTANSPRÜCHE

Die vorliegende europäische Patentanmeldung enthielt bei ihrer Einreichung mehr als zehn Patentansprüche.

☐ Nur ein Teil der Anspruchsgebühren wurde innerhalb der vorgeschriebenen Frist entrichtet. Der vorliegende europäische Recherchenbericht wurde für die ersten zehn sowie für jene Patentansprüche erstellt, für die Anspruchsgebühren entrichtet wurden, nämlich Patentansprüche:

☐ Keine der Anspruchsgebühren wurde innerhalb der vorgeschriebenen Frist entrichtet. Der vorliegende europäische Recherchenbericht wurde für die ersten zehn Patentansprüche erstellt.

## MANGELNDE EINHEITLICHKEIT DER ERFINDUNG

Nach Auffassung der Recherchenabteilung entspricht die vorliegende europäische Patentanmeldung nicht den Anforderungen an die Einheitlichkeit der Erfindung und enthält mehrere Erfindungen oder Gruppen von Erfindungen, nämlich:

Siehe Ergänzungsblatt B

☐ Alle weiteren Recherchengebühren wurden innerhalb der gesetzten Frist entrichtet. Der vorliegende europäische Recherchenbericht wurde für alle Patentansprüche erstellt.

☐ Da für alle recherchierbaren Ansprüche die Recherche ohne einen Arbeitsaufwand durchgeführt werden konnte, der eine zusätzliche Recherchengebühr gerechtfertigt hätte, hat die Recherchenabteilung nicht zur Zahlung einer solchen Gebühr aufgefordert.

☐ Nur ein Teil der weiteren Recherchengebühren wurde innerhalb der gesetzten Frist entrichtet. Der vorliegende europäische Recherchenbericht wurde für die Teile der Anmeldung erstellt, die sich auf Erfindungen beziehen, für die Recherchengebühren entrichtet worden sind, nämlich Patentansprüche:

☒ Keine der weiteren Recherchengebühren wurde innerhalb der gesetzten Frist entrichtet. Der vorliegende europäische Recherchenbericht wurde für die Teile der Anmeldung erstellt, die sich auf die zuerst in den Patentansprüchen erwähnte Erfindung beziehen, nämlich Patentansprüche:

1 - 8 (partiell)

**MANGELNDE EINHEITLICHKEIT
DER ERFINDUNG
ERGÄNZUNGSBLATT B**

Nummer der Anmeldung

EP 04 00 9361

Nach Auffassung der Recherchenabteilung entspricht die vorliegende europäische Patentanmeldung nicht den Anforderungen an die Einheitlichkeit der Erfindung und enthält mehrere Erfindungen oder Gruppen von Erfindungen, nämlich:

1. Ansprüche: 1-8(partiell)

    Mittel enthaltend Amidosulfonsäure und Alkylsulfonsäure und Verwendung davon u.a. zur Inaktivierung von Prionen
    ---

2. Ansprüche: 1-8(partiell)

    Mittel enthaltend Amidosulfonsäure und Alkylarylsulfonsäure und Verwendung davon u.a. zur Inaktivierung von Prionen
    ---

3. Ansprüche: 1-8(partiell)

    Mittel enthaltend Amidosulfonsäure und Alkylsulfat und Verwendung davon u.a. zur Inaktivierung von Prionen
    ---

4. Ansprüche: 1-8(partiell)

    Mittel enthaltend Amidosulfonsäure und Alkylethersulfat und Verwendung davon u.a. zur Inaktivierung von Prionen
    ---

5. Ansprüche: 1-8(partiell)

    Mittel enthaltend Amidosulfonsäure und Alkylarylethersulfat und Verwendung davon u.a. zur Inaktivierung von Prionen
    ---

6. Ansprüche: 1-8(Partiell)

    Mittel enthaltend Amidosulfonsäure und Ethylpolyethylenglykolether und Verwendung davon u.a. zur Inaktivierung von Prionen
    ---

7. Ansprüche: 1-8(partiell)

    Mittel enthaltend O-phosphorsäure und Alkylsulfonsäure und Verwendung davon u.a. zur Inaktivierung von Prionen
    ---

8. Ansprüche: 1-8(partiell)

    Mittel enthaltend O-phosphorsäure und Alkylarylsulfonsäure und Verwendung davon u.a. zur Inaktivierung von Prionen
    ---

9. Ansprüche: 1-8(partiell)

**Europäisches
Patentamt**

**MANGELNDE EINHEITLICHKEIT
DER ERFINDUNG
ERGÄNZUNGSBLATT B**

Nach Auffassung der Recherchenabteilung entspricht die vorliegende europäische Patentanmeldung nicht den Anforderungen an die Einheitlichkeit der Erfindung und enthält mehrere Erfindungen oder Gruppen von Erfindungen, nämlich:

Mittel enthaltend O-phosphorsäure und Alkylsulfat und Verwendung davon u.a. zur Inaktivierung von Prionen
---

10. Ansprüche: 1-8(partiell)

Mittel enthaltend O-phosphorsäure und Alkylethersulfat und Verwendung davon u.a. zur Inaktivierung von Prionen
---

11. Ansprüche: 1-8(partiell)

Mittel enthaltend O-phosphorsäure und Alkylarylethersulfat und Verwendung davon u.a. zur Inaktivierung von Prionen
---

12. Ansprüche: 1-8(partiell)

Mittel enthaltend O-phosphorsäure und Alkylpolyethylenglykolether und Verwendung davon u.a. zur Inaktivierung von Prionen
---

13. Ansprüche: 1-8(partiell)

Mittel enthaltend Ameisensäure und Alkylsulfonsäure und Verwendung davon u.a. zur Inaktivierung von Prionen
---

14. Ansprüche: 1-8(partiell)

Mittel enthaltend Ameisensäure und Alkylarylsulfonsäure und Verwendung davon u.a. zur Inaktivierung von Prionen
---

15. Ansprüche: 1-8(partiell)

Mittel enthaltend Ameisensäure und Alkylsulfat und Verwendung davon u.a. zur Inaktivierung von Prionen
---

16. Ansprüche: 1-8(partiell)

Mittel enthaltend Ameisensäure und Alkylethersulfat und Verwendung davon u.a. zur Inaktivierung von Prionen
---

17. Ansprüche: 1-8(partiell)

Mittel enthaltend Ameisensäure und Alkylarylethersulfat und Verwendung davon u.a. zur Inaktivierung von Prionen

**Europäisches**
**Patentamt**

**MANGELNDE EINHEITLICHKEIT**
**DER ERFINDUNG**
**ERGÄNZUNGSBLATT B**

**Nummer der Anmeldung**

**EP 04 00 9361**

Nach Auffassung der Recherchenabteilung entspricht die vorliegende europäische Patentanmeldung nicht den Anforderungen an die Einheitlichkeit der Erfindung und enthält mehrere Erfindungen oder Gruppen von Erfindungen, nämlich:

---

18. Ansprüche: 1-8(partiell)

    Mittel enthaltend Ameisensäure und Alkylpolyethylenglykolether und Verwendung davon u.a. zur Inaktivierung von Prionen

    ---

19. Ansprüche: 1-8(partiell)

    Mittel enthaltend Glyoxylsäure und Alkylsulfonsäure und Verwendung davon u.a. zur Inaktivierung von Prionen

    ---

20. Ansprüche: 1-8(partiell)

    Mittel enthaltend Glyoxylsäure und Alkylarylsulfonsäure und Verwendung davon u.a. zur Inaktivierung von Prionen

    ---

21. Ansprüche: 1-8(partiell)

    Mittel enthaltend Glyoxylsäure und Alkylsulfat und Verwendung davon u.a. zur Inaktivierung von Prionen

    ---

22. Ansprüche: 1-8(partiell)

    Mittel enthaltend Glyoxylsäure und Alkylethersulfat und Verwendung davon u.a. zur Inaktivierung von Prionen

    ---

23. Ansprüche: 1-8(partiell)

    Mittel enthaltend Glyoxylsäure und Alkylarylethersulfat und Verwendung davon u.a. zur Inaktivierung von Prionen

    ---

24. Ansprüche: 1-8(partiell)

    Mittel enthaltend Glyoxylsäure und Alkylpolyethylenglykolether und Verwendung davon u.a. zur Inaktivierung von Prionen

    ---

25. Ansprüche: 1-8(partiell)

    Mittel enthaltend Essigsäure und Alkylsulfonsäure und Verwendung davon u.a. zur Inaktivierung von Prionen

    ---

**Europäisches Patentamt**

**MANGELNDE EINHEITLICHKEIT DER ERFINDUNG ERGÄNZUNGSBLATT B**

**Nummer der Anmeldung**

EP 04 00 9361

Nach Auffassung der Recherchenabteilung entspricht die vorliegende europäische Patentanmeldung nicht den Anforderungen an die Einheitlichkeit der Erfindung und enthält mehrere Erfindungen oder Gruppen von Erfindungen, nämlich:

26. Ansprüche: 1-8(partiell)

Mittel enthaltend Essigsäure und Alkylarylsulfonsäure und Verwendung davon u.a. zur Inaktivierung von Prionen
---

27. Ansprüche: 1-8(partiell)

Mittel enthaltend Essigsäure und Alkylsulfat und Verwendung davon u.a. zur Inaktivierung von Prionen
---

28. Ansprüche: 1-8(partiell)

Mittel enthaltend Essigsäure und Alkylethersulfat und Verwendung davon u.a. zur Inaktivierung von Prionen
---

29. Ansprüche: 1-8(partiell)

Mittel enthaltend Essigsäure und Alkylarylethersulfat und Verwendung davon u.a. zur Inaktivierung von Prionen
---

30. Ansprüche: 1-8(partiell)

Mittel enthaltend Essigsäure und Alkylpolyethylenglykolether und Verwendung davon u.a. zur Inaktivierung von Prionen
---

31. Ansprüche: 1-8(partiell)

Mittel enthaltend Propionsäure und Alkylsulfonsäure und Verwendung davon u.a. zur Inaktivierung von Prionen
---

32. Ansprüche: 1-8(partiell)

Mittel enthaltend Propionsäure und Alkylarylsulfonsäure und Verwendung davon u.a. zur Inaktivierung von Prionen
---

33. Ansprüche: 1-8(partiell)

Mittel enthaltend Propionsäure und Alkylsulfat und Verwendung davon u.a. zur Inaktivierung von Prionen
---

34. Ansprüche: 1-8(partiell)

**Europäisches Patentamt**

**MANGELNDE EINHEITLICHKEIT DER ERFINDUNG ERGÄNZUNGSBLATT B**

**Nummer der Anmeldung**

EP 04 00 9361

Nach Auffassung der Recherchenabteilung entspricht die vorliegende europäische Patentanmeldung nicht den Anforderungen an die Einheitlichkeit der Erfindung und enthält mehrere Erfindungen oder Gruppen von Erfindungen, nämlich:

Mittel enthaltend Propionsäure und Alkylethersulfat und Verwendung davon u.a. zur Inaktivierung von Prionen

---

35. Ansprüche: 1-8(partiell)

Mittel enthaltend Propionsäure und Alkylarylethersulfat und Verwendung davon u.a. zur Inaktivierung von Prionen

---

36. Ansprüche: 1-8(partiell)

Mittel enthaltend Propionsäure und Alkylpolyethylenglykolether und Verwendung davon u.a. zur Inaktivierung von Prionen

---

37. Ansprüche: 1-8(partiell)

Mittel enthaltend Milchsäure und Alkylsulfonsäure und Verwendung davon u.a. zur Inaktivierung von Prionen

---

38. Ansprüche: 1-8(partiell)

Mittel enthaltend Milchsäure und Alkylarylsulfonsäure und Verwendung davon u.a. zur Inaktivierung von Prionen

---

39. Ansprüche: 1-8(partiell)

Mittel enthaltend Milchsäure und Alkylsulfat und Verwendung davon u.a. zur Inaktivierung von Prionen

---

40. Ansprüche: 1-8(partiell)

Mittel enthaltend Milchsäure und Alkylethersulfat und Verwendung davon u.a. zur Inaktivierung von Prionen

---

41. Ansprüche: 1-8(partiell)

Mittel enthaltend Milchsäure und Alkylarylethersulfat und Verwendung davon u.a. zur Inaktivierung von Prionen

---

42. Ansprüche: 1-8(partiell)

Mittel enthaltend Milchsäure und Alkylpolyethylenglykolether und Verwendung davon u.a. zur Inaktivierung von Prionen

**Europäisches Patentamt**

**MANGELNDE EINHEITLICHKEIT DER ERFINDUNG ERGÄNZUNGSBLATT B**

Nummer der Anmeldung

EP 04 00 9361

Nach Auffassung der Recherchenabteilung entspricht die vorliegende europäische Patentanmeldung nicht den Anforderungen an die Einheitlichkeit der Erfindung und enthält mehrere Erfindungen oder Gruppen von Erfindungen, nämlich:

---

43. Ansprüche: 1-8(partiell)

> Mittel enthaltend Äpfelsäure und Alkylsulfonsäure und Verwendung davon u.a. zur Inaktivierung von Prionen
> ---

44. Ansprüche: 1-8(partiell)

> Mittel enthaltend Äpfelsäure und Alkylarylsulfonsäure und Verwendung davon u.a. zur Inaktivierung von Prionen
> ---

45. Ansprüche: 1-8(partiell)

> Mittel enthaltend Äpfelsäure und Alkylsulfat und Verwendung davon u.a. zur Inaktivierung von Prionen
> ---

46. Ansprüche: 1-8(partiell)

> Mittel enthaltend Äpfelsäure und Alkylethersulfat und Verwendung davon u.a. zur Inaktivierung von Prionen
> ---

47. Ansprüche: 1-8(partiell)

> Mittel enthaltend Äpfelsäure und Alkylarylethersulfat und Verwendung davon u.a. zur Inaktivierung von Prionen
> ---

48. Ansprüche: 1-8(partiell)

> Mittel enthaltend Äpfelsäure und Alkylpolyethylenglykolether und Verwendung davon u.a. zur Inaktivierung von Prionen
> ---

49. Ansprüche: 1-8(partiell)

> Mittel enthaltend Weinsäure und Alkylsulfonsäure und Verwendung davon u.a. zur Inaktivierung von Prionen
> ---

50. Ansprüche: 1-8(partiell)

> Mittel enthaltend Weinsäure und Alkylarylsulfonsäure und Verwendung davon u.a. zur Inaktivierung von Prionen
> ---

51. Ansprüche: 1-8(partiell)

# EP 1 588 721 A1

**Europäisches
Patentamt**

**MANGELNDE EINHEITLICHKEIT
DER ERFINDUNG
ERGÄNZUNGSBLATT B**

**Nummer der Anmeldung**

EP 04 00 9361

Nach Auffassung der Recherchenabteilung entspricht die vorliegende europäische Patentanmeldung nicht den Anforderungen an die Einheitlichkeit der Erfindung und enthält mehrere Erfindungen oder Gruppen von Erfindungen, nämlich:

Mittel enthaltend Weinsäure und Alkylsulfat und Verwendung davon u.a. zur Inaktivierung von Prionen

---

52. Ansprüche: 1-8(partiell)

Mittel enthaltend Weinsäure und Alkylethersulfat und Verwendung davon u.a. zur Inaktivierung von Prionen

---

53. Ansprüche: 1-8(partiell)

Mittel enthaltend Weinsäure und Alkylarylethersulfat und Verwendung davon u.a. zur Inaktivierung von Prionen

---

54. Ansprüche: 1-8(partiell)

Mittel enthaltend Weinsäure und Alkylpolyethylenglykolether und Verwendung davon u.a. zur Inaktivierung von Prionen

---

55. Ansprüche: 1-8(partiell)

Mittel enthaltend Citronensäure und Alkylsulfonsäure und Verwendung davon u.a. zur Inaktivierung von Prionen

---

56. Ansprüche: 1-8(partiell)

Mittel enthaltend Citronensäure und Alkylarylsulfonsäure und Verwendung davon u.a. zur Inaktivierung von Prionen

---

57. Ansprüche: 1-8(partiell)

Mittel enthaltend Citronensäure und Alkylsulfat und Verwendung davon u.a. zur Inaktivierung von Prionen

---

58. Ansprüche: 1-8(partiell)

Mittel enthaltend Citronensäure und Alkylethersulfat und Verwendung davon u.a. zur Inaktivierung von Prionen

---

59. Ansprüche: 1-8(partiell)

Mittel enthaltend Citronensäure und Alkylarylethersulfat und Verwendung davon u.a. zur Inaktivierung von Prionen

# EP 1 588 721 A1

**Europäisches**
**Patentamt**

**MANGELNDE EINHEITLICHKEIT**
**DER ERFINDUNG**
**ERGÄNZUNGSBLATT B**

**Nummer der Anmeldung**

**EP 04 00 9361**

Nach Auffassung der Recherchenabteilung entspricht die vorliegende europäische Patentanmeldung nicht den Anforderungen an die Einheitlichkeit der Erfindung und enthält mehrere Erfindungen oder Gruppen von Erfindungen, nämlich:

---

60. Ansprüche: 1-8(partiell)

Mittel enthaltend Citronensäure und Alkylpolyethylenglykolether und Verwendung davon u.a. zur Inaktivierung von Prionen

---

61. Ansprüche: 1-8(partiell)

Mittel enthaltend Glyconsäure und Alkylsulfonsäure und Verwendung davon u.a. zur Inaktivierung von Prionen

---

62. Ansprüche: 1-8(partiell)

Mittel enthaltend Glyconsäure und Alkylarylsulfonsäure und Verwendung davon u.a. zur Inaktivierung von Prionen

---

63. Ansprüche: 1-8(partiell)

Mittel enthaltend Glyconsäure und Alkylsulfat und Verwendung davon u.a. zur Inaktivierung von Prionen

---

64. Ansprüche: 1-8(partiell)

Mittel enthaltend Glyconsäure und Alkylethersulfat und Verwendung davon u.a. zur Inaktivierung von Prionen

---

65. Ansprüche: 1-8(partiell)

Mittel enthaltend Glyconsäure und Alkylarylethersulfat und Verwendung davon u.a. zur Inaktivierung von Prionen

---

66. Ansprüche: 1-8(partiell)

Mittel enthaltend Glyconsäure und Alkylpolyethylenglykolether und Verwendung davon u.a. zur Inaktivierung von Prionen

---

67. Ansprüche: 1-8(partiell)

Mittel enthaltend Cyclohexansulfaminsäure und Alkylsulfonsäure und Verwendung davon u.a. zur Inaktivierung von Prionen

**EP 1 588 721 A1**

Europäisches
Patentamt

**MANGELNDE EINHEITLICHKEIT**
**DER ERFINDUNG**
**ERGÄNZUNGSBLATT B**

Nummer der Anmeldung

EP 04 00 9361

Nach Auffassung der Recherchenabteilung entspricht die vorliegende europäische Patentanmeldung nicht den Anforderungen an die Einheitlichkeit der Erfindung und enthält mehrere Erfindungen oder Gruppen von Erfindungen, nämlich:

- - -

68. Ansprüche: 1-8(partiell)

Mittel enthaltend Cyclohexansulfaminsäure und Alkylarylsulfonsäure und Verwendung davon u.a. zur Inaktivierung von Prionen

- - -

69. Ansprüche: 1-8(partiell)

Mittel enthaltend Cyclohexansulfaminsäure und Alkylsulfat und Verwendung davon u.a. zur Inaktivierung von Prionen

- - -

70. Ansprüche: 1-8(partiell)

Mittel enthaltend Cyclohexansulfaminsäure und Alkylethersulfat und Verwendung davon u.a. zur Inaktivierung von Prionen

- - -

71. Ansprüche: 1-8(partiell)

Mittel enthaltend Cyclohexansulfaminsäure und Alkylarylethersulfat und Verwendung davon u.a. zur Inaktivierung von Prionen

- - -

72. Ansprüche: 1-8(partiell)

Mittel enthaltend Cyclohexansulfaminsäure und Alkylpolyethylenglykolether und Verwendung davon u.a. zur Inaktivierung von Prionen

- - -

73. Ansprüche: 1-8(partiell)

Mittel enthaltend Benzisothiazolin-2-on-S,S-dioxid und Alkylsulfonsäure und Verwendung davon u.a. zur Inaktivierung von Prionen

- - -

74. Ansprüche: 1-8(partiell)

Mittel enthaltend Benzisothiazolin-2-on-S,S-dioxid und Alkylarylsulfonsäure und Verwendung davon u.a. zur Inaktivierung von Prionen

- - -

75. Ansprüche: 1-8(partiell)

**MANGELNDE EINHEITLICHKEIT
DER ERFINDUNG
ERGÄNZUNGSBLATT B**

Nummer der Anmeldung

EP 04 00 9361

Nach Auffassung der Recherchenabteilung entspricht die vorliegende europäische Patentanmeldung nicht den Anforderungen an die Einheitlichkeit der Erfindung und enthält mehrere Erfindungen oder Gruppen von Erfindungen, nämlich:

```
     Mittel enthaltend Benzisothiazolin-2-on-S,S-dioxid und
     Alkylsulfat und Verwendung davon u.a. zur Inaktivierung von
     Prionen
                              ---

76. Ansprüche: 1-8(partiell)

     Mittel enthaltend Benzisothiazolin-2-on-S,S-dioxid und
     Alkylethersulfat und Verwendung davon u.a. zur Inaktivierung
     von Prionen
                              ---

77. Ansprüche: 1-8(partiell)

     Mittel enthaltend Benzisothiazolin-2-on-S,S-dioxid und
     Alkylarylethersulfat und Verwendung davon u.a. zur
     Inaktivierung von Prionen
                              ---

78. Ansprüche: 1-8(partiell)

     Mittel enthaltend Benzisothiazolin-2-on-S,S-dioxid und
     Alkylpolyethylenglykolether und Verwendung davon u.a. zur
     Inaktivierung von Prionen
                              ---

79. Ansprüche: 1-8(partiell)

     Mittel enthaltend Nitriloessigsäure und Alkylsulfonsäure und
     Verwendung davon u.a. zur Inaktivierung von Prionen
                              ---

80. Ansprüche: 1-8(partiell)

     Mittel enthaltend Nitriloessigsäure und Alkylarylsulfonsäure
     und Verwendung davon u.a. zur Inaktivierung von Prionen
                              ---

81. Ansprüche: 1-8(partiell)

     Mittel enthaltend Nitriloessigsäure und Alkylsulfat und
     Verwendung davon u.a. zur Inaktivierung von Prionen
                              ---

82. Ansprüche: 1-8(partiell)

     Mittel enthaltend Nitriloessigsäure und Alkylethersulfat und
     Verwendung davon u.a. zur Inaktivierung von Prionen
                              ---
```

Europäisches
Patentamt

**MANGELNDE EINHEITLICHKEIT
DER ERFINDUNG
ERGÄNZUNGSBLATT B**

Nummer der Anmeldung

EP 04 00 9361

Nach Auffassung der Recherchenabteilung entspricht die vorliegende europäische Patentanmeldung nicht den Anforderungen an die Einheitlichkeit der Erfindung und enthält mehrere Erfindungen oder Gruppen von Erfindungen, nämlich:

83. Ansprüche: 1-8(partiell)

Mittel enthaltend Nitriloessigsäure und Alkylarylethersulfat und Verwendung davon u.a. zur Inaktivierung von Prionen
---

84. Ansprüche: 1-8(partiell)

Mittel enthaltend Nitriloessigsäure und Alkylpolyethylenglykolether und Verwendung davon u.a. zur Inaktivierung von Prionen
---

85. Ansprüche: 1-8(partiell)

Mittel enthaltend Ethylendiamintetraessigsäure und Alkylsulfonsäure und Verwendung davon u.a. zur Inaktivierung von Prionen
---

86. Ansprüche: 1-8(partiell)

Mittel enthaltend Ethylendiamintetraessigsäure und Alkylarylsulfonsäure und Verwendung davon u.a. zur Inaktivierung von Prionen
---

87. Ansprüche: 1-8(partiell)

Mittel enthaltend Ethylendiamintetraessigsäure und Alkylsulfat und Verwendung davon u.a. zur Inaktivierung von Prionen
---

88. Ansprüche: 1-8(partiell)

Mittel enthaltend Ethylendiamintetraessigsäure und Alkylethersulfat und Verwendung davon u.a. zur Inaktivierung von Prionen
---

89. Ansprüche: 1-8(partiell)

Mittel enthaltend Ethylendiamintetraessigsäure und Alkylarylethersulfat und Verwendung davon u.a. zur Inaktivierung von Prionen
---

90. Ansprüche: 1-8(partiell)

**Europäisches**
**Patentamt**

**MANGELNDE EINHEITLICHKEIT**
**DER ERFINDUNG**
**ERGÄNZUNGSBLATT B**

Nummer der Anmeldung

EP 04 00 9361

Nach Auffassung der Recherchenabteilung entspricht die vorliegende europäische Patentanmeldung nicht den Anforderungen an die Einheitlichkeit der Erfindung und enthält mehrere Erfindungen oder Gruppen von Erfindungen, nämlich:

> Mittel enthaltend Ethylendiamintetraessigsäure und Alkylpolyethylenglykolether und Verwendung davon u.a. zur Inaktivierung von Prionen
>
> ---

91. Ansprüche: 1-8(partiell)

> Mittel enthaltend organisch sustituierte Phosphonsäure und Alkylsulfonsäure und Verwendung davon u.a. zur Inaktivierung von Prionen
>
> ---

92. Ansprüche: 1-8(partiell)

> Mittel enthaltend organisch sustituierte Phosphonsäure und Alkylarylsulfonsäure und Verwendung davon u.a. zur Inaktivierung von Prionen
>
> ---

93. Ansprüche: 1-8(partiell)

> Mittel enthaltend organisch sustituierte Phosphonsäure und Alkylsulfat und Verwendung davon u.a. zur Inaktivierung von Prionen
>
> ---

94. Ansprüche: 1-8(partiell)

> Mittel enthaltend organisch sustituierte Phosphonsäure und Alkylethersulfat und Verwendung davon u.a. zur Inaktivierung von Prionen
>
> ---

95. Ansprüche: 1-8(partiell)

> Mittel enthaltend organisch sustituierte Phosphonsäure und Alkylarylethersulfat und Verwendung davon u.a. zur Inaktivierung von Prionen
>
> ---

96. Ansprüche: 1-8(partiell)

> Mittel enthaltend organisch sustituierte Phosphonsäure und Alkylpolyethylenglykolether und Verwendung davon u.a. zur Inaktivierung von Prionen
>
> ---

**ANHANG ZUM EUROPÄISCHEN RECHERCHENBERICHT**
**ÜBER DIE EUROPÄISCHE PATENTANMELDUNG NR.**    EP 04 00 9361

In diesem Anhang sind die Mitglieder der Patentfamilien der im obengenannten europäischen Recherchenbericht angeführten
Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

08-11-2004

| Im Recherchenbericht angeführtes Patentdokument | Datum der Veröffentlichung | Mitglied(er) der Patentfamilie | Datum der Veröffentlichung |
|---|---|---|---|
| US 2004052833 A1 | 18-03-2004 | US 2003004312 A1 | 02-01-2003 |
| | | US 2002041859 A1 | 11-04-2002 |
| | | US 6322802 B1 | 27-11-2001 |
| | | US 6331296 B1 | 18-12-2001 |
| | | US 6214366 B1 | 10-04-2001 |
| | | US 6221614 B1 | 24-04-2001 |
| | | US 5891641 A | 06-04-1999 |
| | | AU 2002367715 A1 | 29-09-2003 |
| | | EP 1414297 A2 | 06-05-2004 |
| | | WO 03077835 A2 | 25-09-2003 |
| | | US 2004127558 A1 | 01-07-2004 |
| | | US 2004127559 A1 | 01-07-2004 |
| | | AU 3299401 A | 07-08-2001 |
| | | EP 1251737 A2 | 30-10-2002 |
| | | WO 0154736 A2 | 02-08-2001 |
| | | AU 771547 B2 | 25-03-2004 |
| | | AU 5044100 A | 18-12-2000 |
| | | BR 0011055 A | 21-05-2002 |
| | | CA 2375237 A1 | 07-12-2000 |
| | | EP 1187622 A1 | 20-03-2002 |
| | | JP 2003500169 T | 07-01-2003 |
| | | MX PA01012357 A | 02-09-2002 |
| | | NZ 515607 A | 25-07-2003 |
| | | WO 0072851 A1 | 07-12-2000 |
| | | US 2002041862 A1 | 11-04-2002 |
| | | US 6419916 B1 | 16-07-2002 |
| | | AU 768032 B2 | 27-11-2003 |
| | | AU 2589800 A | 07-08-2000 |
| | | BR 9916932 A | 30-10-2001 |
| | | CA 2363846 A1 | 27-07-2000 |
| | | CN 1357109 T | 03-07-2002 |
| | | EP 1145013 A2 | 17-10-2001 |
| | | JP 2002539081 T | 19-11-2002 |
| | | WO 0043782 A2 | 27-07-2000 |
| | | US 2003180706 A1 | 25-09-2003 |
| | | US 2004053335 A1 | 18-03-2004 |
| | | US 6620629 B1 | 16-09-2003 |
| | | US 2001005578 A1 | 28-06-2001 |
| | | AT 244889 T | 15-07-2003 |
| | | AU 753331 B2 | 17-10-2002 |
| | | AU 2660299 A | 06-09-1999 |
| | | BR 9908059 A | 08-01-2002 |
| | | CA 2318477 A1 | 26-08-1999 |
| | | DE 69909442 D1 | 14-08-2003 |
| | | DE 69909442 T2 | 22-04-2004 |
| | | DE 1057022 T1 | 25-10-2001 |

Für nähere Einzelheiten zu diesem Anhang : siehe Amtsblatt des Europäischen Patentamts, Nr.12/82

**ANHANG ZUM EUROPÄISCHEN RECHERCHENBERICHT
ÜBER DIE EUROPÄISCHE PATENTANMELDUNG NR.**  EP 04 00 9361

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

08-11-2004

| Im Recherchenbericht angeführtes Patentdokument | Datum der Veröffentlichung | Mitglied(er) der Patentfamilie | Datum der Veröffentlichung |
|---|---|---|---|
| US 2004052833      A1 | | EP      1057022 A1<br>ES      2203073 T3<br>JP   2003519357 T<br>WO      9942829 A1 | 06-12-2000<br>01-04-2004<br>17-06-2003<br>26-08-1999 |
| US 6162371       A | 19-12-2000 | AU       741509 B2<br>AU      2002899 A<br>CA      2316358 A1<br>EP      1056828 A1<br>WO      9932596 A1 | 06-12-2001<br>12-07-1999<br>01-07-1999<br>06-12-2000<br>01-07-1999 |
| US 4247408       A | 27-01-1981 | JP      1194611 C<br>JP     54158409 A<br>JP     58026399 B<br>DE      2922345 A1<br>ES       481297 A1<br>GB      2022126 A ,B<br>MX       150531 A | 12-03-1984<br>14-12-1979<br>02-06-1983<br>13-12-1979<br>16-08-1980<br>12-12-1979<br>22-05-1984 |
| US 5280042       A | 18-01-1994 | US      5143720 A<br>AU      9121191 A<br>CA      2096837 A1<br>EP      0561959 A1<br>JP      6502868 T<br>WO      9209260 A1 | 01-09-1992<br>25-06-1992<br>29-05-1992<br>29-09-1993<br>31-03-1994<br>11-06-1992 |
| GB 896159        A | 09-05-1962 | KEINE | |

EPO FORM P0461

Für nähere Einzelheiten zu diesem Anhang : siehe Amtsblatt des Europäischen Patentamts, Nr.12/82